# EUROPEAN PATENT SPECIFICATION

(11) **EP 2 450 064 B1**
(45) Date of publication and mention of the grant of the patent: **09.04.2014**
(21) Application number: 10188032.6
(22) Date of filing: 19.10.2010
(51) Int. Cl.: A61L 24/02, A61L 27/12

(54) **Bone Cement Formula and Bioresorbable Hardened Bone Cement Composites Prepared with the Same**
Knochenzementformel und damit hergestellte bioresorbierbare gehärtete Knochenzementverbundstoffe
Formulation de ciment osseux et composites durcis de ciment osseux préparés avec cette formulation

(43) Date of publication of application: 09.05.2012
(73) Proprietor: National Cheng Kung University, Tainan City 701 (TW)
(72) Inventor: Lin, Jiin-Huey Chern, Winnetka, IL 60093 (US); Ju, Chien-Ping, Kansas City, MO 64137 (US)
(74) Representative: Grünecker, Kinkeldey, Stockmair & Schwanhäusser

(56) References cited:
- WO-A2-03/024316
- US-A- 4 612 053
- US-A1- 2007 178 171

## Description

### Field

The exemplary embodiment(s) of the present invention relates to bone repairing substance for medicaments. More specifically, the exemplary embodiment(s) of the present invention relates to a bone cement formula.

### Background

Bone cement compositions are widely used in bonding, filling, and/or repairing damaged natural bone. Bone cement is typically used in orthopedic, dental procedures, and/or other medicinal applications. Despite many advantages such as excellent biocompatibility, superior osteoconductivity, and enhanced mechanical or physical strength, the majority of calcium phosphates exhibit clinically low bioresorption rates. On the other hand, the compound of calcium sulfates also illustrate higher dissolution rates, which, for many applications, is typically too high of dissolution rate for allowing new bone cells to quickly grow in a bone cavity. The composite including calcium sulfates generally has lower mechanical and/or physical strengths than calcium phosphates.

US2007/178171 A1 discloses brushite forming bone cements comprising the powders beta-tricalcium phosphate (TCP), monocalcium phosphate monohydrate (MCP) and calcium sulphate hemihydrate (CSH). Wherein, at least 50% of the composition is a sulphate source. The liquid can comprise salts.

WO03/024316 A2 discloses a bone cement comprising the powders: TTCP, DCP and up to 50% CSH. However, the liquid to powder ratio is not 0.20-0.50 ml/g and the liquid does not comprise ammonium ions. Moreover, the ratio of TTCP:DCP is not revealed.

Another problem associated with conventional bone cement paste is prolong setting time whereby it hampers applicability to various applications. For example, calcium phosphate (CPC) paste, which is described in US patent No.: 4,612,053, typically requires prolong setting time.

### Summary

Embodiment(s) of the present invention discloses a method of providing a calcium phosphate-calcium sulfate composite that exhibits enhanced strength, excellent biocompatibility, superior osteoconductivity, appropriate and adjustable bioresorption rate.

The objectives of embodiments are to provide a bone cement formula, a bone cement paste, a hardened bone cement composite from paste, a hardened bone cement composite with enhanced strength by pressurizing the paste while leaking solution from the paste, and porous hardened bone cement composite from the paste.

The embodiments of the present invention provides methods for providing a bone cement formula, bone cement paste, hardened bone cement composite, hardened bone cement composite with enhanced strength, and porous hardened bone cement composite.

The present invention provides a bone cement formula which includes a powder component and a setting liquid component with a liquid to powder ratio of 0.20 cc/g to 0.50 cc/g (cc is cubic centimeter, g is gram), preferably 0.25 cc/g to 0.35 cc/g. The powder component, in one aspect, includes a calcium sulfate source and a calcium phosphate source. The setting liquid component includes ammonium ion (NH₄⁺) in a concentration of about 0.5 M to 4 M. The calcium phosphate source includes tetracalcium phosphate (TTCP) and dicalcium phosphate in a molar ratio of TTCP to dicalcium phosphate of about 0.5 to about 2.5, preferably about 1.0, and the calcium sulfate source is calcium sulfate hemihydrate (CSH), calcium sulfate dehydrate (CSD), or anhydrous calcium sulfate, and preferably, CSH.

It should be noted that, for different medical indications, the required resorption rates can be different. As such, more desirable cement should be able to provide a range of bone resorption rates without substantially changing its primary formula, performance and working/setting times. For example, a resorption rate of implanted hardened cement composite can be adjustable due to co-existence of a calcium sulfate source and a calcium phosphate source. Our animal study demonstrates that the resorption rate of our hardened sulfate-phosphate cement composite can be adjusted by adjusting the sulfate/phosphate ratio.

The weight ratio of the calcium sulfate source of the powder component is 5% to 55%, based on the total weight of the calcium sulfate source and the calcium phosphate source powder.

As illustrated or demonstrated in Control Examples 1-4, detailed discussion in the section of Experiential Procedures below, the combination of TTCP, DCPA and CSH is essential. As TTCP, DCPA, and CSH can be combined or mixed in a certain weight-ratio range, various unique and non-replaceable results are obtained. On the other hand, various experiments of mixtures of two compounds, such as TTCP/CSH and DCPA/CSH, result unsatisfactory results.

Although ammonium is generally considered as a rather toxic component, the experiment, shown below, illustrates that ammonium provides not only a cytotoxically acceptable cement formula, but also a cement formula with unprecedented performance. When concentration of ammonium ions is too low, the cement paste can be either dispersed right upon contact with liquid, such as water or body fluid (i.e., blood), or has an initial mechanical strength that is too low to maintain cement paste integrity which can cause premature fracture of cement paste. On the other hand, when ammonium ion concentration is too high, the cement paste becomes too toxic to be used as an implant.

In one embodiment, the setting liquid component comprises ammonium ion (NH₄⁺) in a concentration of about 1.0 M to 2.0 M, and more preferably about 1.2 M.

The setting liquid component, in one example, is a solution of NH₄H₂PO₄, (NH₄)₂HPO₄, (NH₄)₃PO₄·3H₂O or a mixture of them. Preferably, the setting liquid component is an aqueous solution. Selectively, the setting liquid component further comprises citric acid or tartaric acid dissolved therein. The setting liquid component preferably has a pH of about 7.0 to about 9.0.

In one embodiment, the powder component comprises a pore-forming agent which is to be dissolved in a solution when hardened bone cement composite is immersed in the solution. Preferably, the pore-forming agent is selected from the group consisting of LiCl, KCl, NaCl, MgCl₂, CaCl₂, NalO₃, Kl, Na₃PO₄, K₃PO₄, Na₂CO₃, amino acid-sodium salt, amino acid-potassium salt, glucose, polysaccharide, fatty acid-sodium salt, fatty acid-potassium salt, potassium bitartrate (KHC₄H₄O₆) potassium carbonate, potassium gluconate (KC₆H₁₁O₇), potassium-sodium tartrate (KNaC₄H₄O₆·4H₂O), potassium sulfate (K₂SO₄), sodium sulfate, sodium lactate and mannitol. The amount of the pore-forming agent used is proportional to the porosity of the hardened bone cement composite to be achieved.

In one embodiment, the calcium phosphate source is a mixture of TTCP and DCPA. The bone cement formula, in one aspect, allows bone cement paste having a desirable working time and a setting time, so that the operator has a sufficient period of time to fill the hole or cavity with the paste before the paste becomes hardened. It should be noted that filled paste will develop minimum strength required by the treatment within an acceptable short period of time.

The hardened bone cement composite, in one embodiment, possesses a low toxicity whereby it is safe to be applied to a patient, for example. Note that hardened bone cement has a characteristic of high initial strength with an improved bioresorbable rate.

Additional features and benefits of the present invention will become apparent from the detailed description, figures and claims set forth below.

### Detailed Description

Embodiments of the present invention are described herein in the context of method, formula, system, and/or process for preparing a hardened bone cement composite having an enhanced bioresorbable rate for medicaments. Those of ordinary skills in the art will realize that the following detailed description of the embodiment(s) is illustrative only and is not intended to be in any way limiting. Other embodiments of the present invention will readily suggest themselves to such skilled persons having the benefit of this disclosure.

References to "one embodiment," "an embodiment," "example embodiment," "various embodiments," "exemplary embodiment," "one aspect," "an aspect," "exemplary aspect," "various aspects," etc., indicate that the embodiment(s) of the invention so described may include a particular feature, structure, or characteristic, but not every embodiment necessarily includes the particular feature, structure, or characteristic. Further, repeated use of the phrase "in one embodiment" does not necessarily refer to the same embodiment, although it may.

In the interest of clarity, not all of the routine features of the implementations and/or processes described herein are shown and described. It will, of course, be appreciated that in the development of any such actual implementation, numerous implementation-specific decisions will be made in order to achieve the developer's specific goals, such as compliance with application- and business-related constraints, and that these specific goals will vary from one implementation to another and from one developer to another. Moreover, it will be appreciated that such a development effort might be complex and time-consuming, but would nevertheless be a routine undertaking for those of ordinary skills in the art having the benefit of this disclosure.

Embodiment(s) of the present invention is bioresorbable bone cement applicable to various medical fields, such as orthopedic, spinal, and root canal surgeries. Characteristics or properties of the bioresorbable bone cement or formula have convenient working environment(s) and setting times to form a hardened block with high strength, excellent biocompatibility and superior osteoconductivity, and adjustable (or flexible) bioresorption rate.

To prepare or make the bioresorbable bone cement having properties of flexible (or convenient) work environment (or time) and setting times to form hardened bone cement composites with desirable strength and biological compatibility, one embodiment of the present invention discloses a bone cement formula which is be described more details below. In one aspect, a method or process for preparing hardened bone cement composite involves producing bone cement paste and placing the paste in an environment where the paste can set.

A process for preparing bone cement paste, in one embodiment, comprises mixing powder component with setting liquid component by a mixing mechanism such as agitation. The powder component, for example, may include mixture of calcium sulfate source and calcium phosphate source. Alternatively, calcium sulfate source and calcium phosphate source can be separate powders. In this case, calcium sulfate source and calcium phosphate source are combined first to form a power mixture prior to mixing with setting liquid component.

The calcium sulfate source and calcium phosphate source discussed earlier can be tetracalcium phosphate (TTCP) and/or dicalcium phosphate anhydrous (DCPA) powders. It should be noted that other types of sources can be used as long as they have similar chemical properties or characteristics as TTCP and/or DCPA.

The bone cement paste, in one embodiment, becomes hard or cured within a period of setting time under an atmosphere environment or an environment surrounded by body fluid such as blood. During an operation, an operator or doctor places bone cement paste into a hole or cavity at a damaged bone via a suitable tool through an incision. For example, for an orthopedic, spinal or root canal treatment, when bone cement paste becomes or cures into hardened bone cement composite in-situ, the hardened bone cement will be resorbed by the subject body over time in accordance with a predefined bioresorption rate. Depending on applications, bone cement paste, in one embodiment, can be shaped into a rigid or semi-rigid block of bone cement composite before it is implanted in the subject body to repair damaged parts such as bones or teeth.

The bone cement paste, in one embodiment, can be injected into a bone hole or cavity with an orthopedic paste delivering tool such as a conventional medical instrument described in US 7,325,702 B2 or shaped through a mold, in which the paste will form a block of hardened bone cement composite. It should be noted that an orthopedic delivering tool is able to continually deliver the paste into a bone cavity until the cavity is filled.

Depending on applications, a dense block of cement can be formed if the powder component does not contain the appropriate pore-forming agents. The dense block, for example, can be formed by pressurizing the bone cement paste in a mold before the paste sets in order to drain or remove a portion of liquid from the paste whereby a liquid/powder ratio of the paste decreases. In one aspect, the pressure applied to the paste at the mold has a range from approximately 1 megapascal ("MPa") to 500 MPa, preferably from 100 MPa to 500 MPa. It is noted that the dense block has a superior compressive strength which can be used as a medical implant. It should be further noted that a rigid or solid dense block of calcium phosphate cement is impregnated with an impregnating liquid for a predefined period of time, so that overall compressive strength of the resulting impregnated block is increased compared to a block which has not undergone such impregnating treatment. The impregnating liquid, in one embodiment, is phosphate-containing solution. Exemplary aqueous solution may include, but not limited to, (NH₄)₃PO₄, (NH₄)₂HPO₄, NH₄H₂PO₄, K₃PO₄, K₂HPO₄, KH₂PO_{4,} Na₃PO₄, Na₂HPO₄ NaH₂PO₄, or H₃PO₄. The phosphate-containing solution, in one example, has a phosphate concentration of about 0.1 M to about 6 M, preferably from about 1 M to about 3 M.

A porous block can be utilized as a tissue-engineered scaffold when the powder component of the bone cement formula contains pore-forming agent. The pore-forming agent can be removed from a molded block by immersing the molded block in immersing liquid so that the pore-forming agent is dissolved in the immersing liquid. The pore-forming agent may be added during the mixing of the powder component and the setting liquid component, or may be added to the resultant paste before it is placed in a mold. The immersing liquid, for example, can be an acidic aqueous solution, a basic aqueous solution, a physiological solution, an organic solvent or substantially pure water. In one embodiment, the immersing liquid is the same as the above-mentioned impregnating liquid. In one embodiment, the immersing liquid is waster. In one aspect, the porous block has a porosity of 50-90 vol%. The dense block or porous block prepared in accordance with the bone cement formula, in one embodiment, can be deposited within living cells, a growth factor and/or a drug by impregnating the block in a suspension of living cells or a solution of the growth factor and/or drug. The dense block and the porous block prepared in the present invention may be further broken up into pellets for other medical applications.

The following examples via experimental procedures are illustrative and are intended to demonstrate embodiments of the present invention, which, however, should not be taken to limit the embodiments of the invention to the specific embodiments, but are for explanation and understanding only, since numerous modifications and variations will be apparent to those skilled persons in this art.

### Experimental procedures

### Abbreviation

TTCP: tetracalcium phosphate
DCPA: dicalcium phosphate anhydrous
CSH: calcium sulfate hemihydrate
WT: Working time
ST: Setting time
L/P ratio: Liquid/powder ratio
CS: Compressive strength

### Symbols used in tables

#: Paste cannot be formed by mixing powder and liquid for 2 minutes.
*: After being removed from the mold (30 minutes from mixing powder and liquid), the hardened cement blocks collapses and disintegrates into powder form when it is immersed in Hanks' solution for 1 day.
□: Hardened cement blocks are broken (fractured/cracked but not dispersed into powder form) after being immersed in Hanks' solution for 1 day.

### Preparation of TTCP powder

The TTCP powder was fabricated in-house from the reaction of dicalcium pyrophosphate (Ca₂P₂O₇) (Sigma Chem. Co., St. Louis, MO, USA) and calcium carbonate (CaCO₃) (Katayama Chem. Co., Tokyo, Japan) using the method suggested by Brown and Epstein [Journal of Research of the National Bureau of Standards- A Physics and Chemistry 6 (1965) 69A 12].

TTCP powder was prepared by mixing Ca₂P₂O₇ powder with CaCO₃ powder uniformly for 12 hours. The mixing ratio of Ca₂P₂O₇ powder to CaCO₃ powder was 1:1.27 (weight ratio) and the powder mixture was heated to 1400°C to allow two powders to react to form TTCP.

### Preparation of a TTCP/DCPA/CSH composite paste

Appropriate amounts of TTCP and DCPA powders were uniformly mixed in a ball miller, followed by uniformly mixing with appropriate amount of CSH powder. The resultant TTCP/DCPA/CSH mixed powders were mixed uniformly with a desirable setting solution (e.g., 0.6M (NH₄)₂HPO₄) at a desirable L/P ratio (e.g., 0.28 mL/g (cc/g)) to form a TTCP/DCPA/CSH paste.

### Chemicals used for the study

| Chemical | Formula | Manufacturer | Location |
|---|---|---|---|
| Tetracalcium phosphate (TTCP) | Ca₄(PO₄)₂O | Fabricated in-house | Taiwan |
| Dicalcium phosphate anhydrous (DCPA) | CaHPO₄ | ACROS | New jersey, USA |
| Calcium sulfate hemihydrate (CSH) | CaSO₄□1/2H₂O | Showa | Tokyo, Japan |
| Diammonium hydrogen phosphate | (NH₄)₂HPO₄ | Showa | Tokyo, Japan |
| Diammonium dihydrogen phosphate | NH₄H₂PO₄ | Showa | Tokyo, Japan |
| Dipotassium hydrogen phosphate | K₂HPO₄ | Showa | Tokyo, Japan |
| Tartaric acid | C₂H₂(OH)₂(COOH)₂ | Katayama | Osaka, Japan |
| Citric acid | C₆H₈O₇ | Pantreac | Barcelona, Spain |
| Malic acid | C₂H₃(OH)(COOH)₂ | Pantreac | |

### Compressive strength testing of composite cement

To measure the CS of a hardened cement, after mixing for 1 min, the cement paste was packed in a 6 mm diameter, 12 mm deep cylindrical stainless steel mold under a pressure of 1.4 MPa for 30 min. After being removed from the mold, the hardened cement samples were immersed in Hanks' physiological solution which was maintained at 37°C and agitated daily to help maintain uniform ion concentrations. After immersion, samples were removed from the solution for CS testing while samples are still wet ("test under wet condition"). The CS testing was conducted using a desk-top mechanical tester (Shimadzu AG-10kNX, Tokyo, Japan) at a crosshead speed of 1.0 mm/min. The test method is according to ASTM 451-99a method.

### Working time/setting time measurement

The working time of cement paste was determined by the time after that the cement paste was no longer workable. The setting time of cement paste was measured according to the standard method set forth in ISO 1566 for dental zinc phosphate cements. The cement is considered set when a 400 gm weight loaded onto a Vicat needle with a 1 mm diameter tip fails to make a perceptible circular indentation on the surface of the cement.

### pH measurement

The early stage (during setting process) variation in pH was determined using a pH meter (Suntex Instruments SP2000, Taipei, Taiwan) that was buried in the cement paste immediately after the powder and setting liquid were mixed. The first reading was taken at 1 minute after mixing. The measurement was continued until the paste nearly becomes set. Readings were taken every 30 seconds until 30 minutes after mixing. After then they were taken every 60 seconds.

The variation in pH value of Hanks' solution in which the cement paste sample was immersed was monitored using the same pH meter. 2 g cement paste was taken after mixing the powder and the setting solution for 5 minutes, and it was immersed in 20 ml Hanks' solution with a pH value of 7.05 for the test. The solution was maintained at 37°C throughout testing and continually stirred to help maintain uniform ion concentrations of the solution.

### Cytotoxicity test

The cytotoxicity test was performed according to ISO 10993-5. The extraction method was used. NIH/3T3 fibroblasts (seeding density 5000 per well) were pre-cultured for 24h in Dulbecco's modified essential medium (DMEM) supplemented with bovine serum (10%) and PSF (1%). An extract was prepared by immersing a hardened cement paste in the culture medium at a ratio of 0.1 (g/ml) at 37°C for 24h and then collecting the liquid by centrifugation. The extract was added to the 96 well microplate (100 µl per well) incubated in a 5% CO₂ humidified atmosphere at 37°C. After 24h, the extract was sucked out and then a mixture of the culture medium (100 µl) and WST-1 (10 µl) was added to the wells and incubated for 1 h at 37°C. Cell viability was measured by using the WST-1 assay. This is a colorimetric assay of mitochondrial dehydrogenase activity where the absorbance at 450 nm is proportional to the amount of dehydrogenase activity in the cell. After 1h incubation, the mixture of medium and WST-1 was transferred to a 96 well microplate and the absorbance at 450 nm was measured with an ELISA reader. Al₂O₃ powder was also assayed as a control. Four bars were tested for each sample (*n*=*4*).

**Cell line information**

| | | |
|---|---|---|
| Cell name | | NIH/3T3 |
| Cell number | | BCRC 60008 |
| Type | | Mouse NIH/Swiss embryo |
| Growth property | | Adherent, 5% CO₂, 37°C |
| Morphology | | Fibroblast |
| Cell culture medium | | 90% Dulbecco's modified Eagle's |
| | medium (DMEM) +10% calf serum(CS) | |
| Freeze medium | | 93% culture medium + 7% DMSO |

### Control 1: TTCP/CSH cement and (NH₄)₂HPO₄ setting solution

**Table 1. TTCP/CSH mixed with 0.25-0.75M (NH₄)₂HPO₄**

| TTCP/CSH (wt ratio) | (NH₄)₂HPO₄ concentration (M) | L/P ratio (cc/g) ml/g | WT (min) | ST (min) | 1 d-CS (MPa) |
|---|---|---|---|---|---|
| 90/10 | 0.25 | 0.28 | - | - | * |
| | | 0.33 | - | - | □ |
| | | 0.35 | | | □ |
| | 0.50 | 0.28 | 7.4±0.6 | 9.0±0.7 | 3.23±0.53 |
| | | 0.33 | 10.3±0.6 | 11.6±0.5 | 2.33±0.56 |
| | | 0.35 | 11.0±0.3 | 12.9±0.2 | 2.40±0.71 |
| | 0.60 | 0.28 | 7.0±0.3 | 8.5±0.5 | 3.67±0.49 |
| | | 0.33 | 10.1±0.5 | 11.5±0.6 | 3.06±0.44 |
| | | 0.35 | 11.9±0.6 | 13.0±0.7 | 3.56±0.74 |
| | 0.75 | 0.28 | 5.8±0.2 | 7.1±0.5 | 4.07±1.02 |
| | | 0.33 | 8.9±0.1 | 11.0±0.2 | 4.84 ±0.59 |
| | | 0.35 | 10.4±0.5 | 12.2±0.2 | 3.74±0.19 |
| 75/25 | 0.25 | 0.28 | - | - | * |
| | | 0.33 | - | - | * |
| | | 0.35 | | | * |
| | 0.50 | 0.28 | 7.5±0.3 | 10.1±0.2 | 4.32±0.11 |
| | | 0.33 | 9.7±0.3 | 11.7±0.3 | 7.01±0.42 |
| | | 0.35 | 10.7±0.3 | 12.5±0.4 | 7.32±0.38 |
| | 0.60 | 0.28 | 6.5±0.3 | 8.2±0.2 | 5.92±0.42 |
| | | 0.33 | 7.1±0.3 | 9.1±0.3 | 7.63±0.28 |
| | | 0.35 | 9.0±0.2 | 10.4±0.1 | 8.32±0.31 |
| | 0.75 | 0.28 | 6.4±0.2 | 8.0±0.1 | 6.64±0.52 |
| | | 0.33 | 7.5±0.3 | 9.0±0.4 | 9.55±0.25 |
| | | 0.35 | 8.3±0.3 | 9.9±0.3 | 10.36±0.27 |
| 65/35 | 0.25 | 0.28 | - | - | * |
| | | 0.33 | | | * |
| | | 0.35 | | | * |
| | 0.50 | 0.28 | 7.1±0.3 | 9.8±0.3 | 3.37±0.27 |
| | | 0.33 | 9.2±0.2 | 11.1±0.3 | 5.84±0.37 |
| | | 0.35 | 10.5±0.3 | 12.2±0.4 | 6.10±0.26 |
| | 0.60 | 0.28 | 4.5±0.4 | 5.3±0.2 | 3.96±0.12 |
| | | 0.33 | 7.0±0.3 | 9.0±0.4 | 5.44±0.14 |
| | | 0.35 | 8.9±0.4 | 9.8±0.3 | 6.56±0.12 |
| | 0.75 | 0.28 | 6.3±0.2 | 7.8±0.2 | 4.41±0.24 |
| | | 0.33 | 7.1±0.2 | 8.4±0.3 | 6.43±0.27 |
| | | 0.35 | 8.2±0.3 | 9.3±0.2 | 7.55±0.34 |

In the cases where the pastes were prepared with 0.25 M (NH₄)₂HPO₄ the molded lumps collapse and disintegrate when they are immersed in Hanks' solution (*), or their compressive strength cannot be measured after being immersed in Hanks' solution for 1 day (□) as shown in Table 1. As to the pastes prepared with higher concentration (NH₄)₂HPO₄ setting solutions, the hardened cement blocks give a very low compressive strength after being immersed in Hanks' solution for 1 day (1d-CS) as shown in Table 1. Apparently the powder component having only TTCP and CSH phases does not give a satisfactory result.

### Control 2: TTCP/CSH cement and NH₄H₂PO₄ setting solution

**Table 2. TTCP/CSH mixed with 0.25-0.75M NH₄H₂PO₄**

| TTCP/CSH (weight ratio) | NH₄H₂PO₄ concentration (M) | L/P ratio (cc/g) ml/g | WT (min) | ST (min) | 1 d-CS (MPa) |
|---|---|---|---|---|---|
| 90/10 | 0.25 | 0.28 | - | - | * |
| | | 0.33 | - | - | * |
| | | 0.35 | - | - | * |
| | 0.50 | 0.28 | 7.4±0.6 | 9.0±0.7 | 3.23±0.53 |
| | | 0.33 | 10.3±0.6 | 11.6±0.5 | 2.33±0.56 |
| | | 0.35 | 11.0±0.3 | 12.9±0.2 | 2.40±0.71 |
| | 0.60 | 0.28 | 7.0±0.3 | 8.5±0.5 | 3.67±0.49 |
| | | 0.33 | 10.1±0.5 | 11.5±0.6 | 3.06±0.44 |
| | | 0.35 | 11.9±0.4 | 13.0±0.7 | 3.56±0.74 |
| | 0.75 | 0.28 | 5.8±0.2 | 7.1±0.5 | 4.07±1.02 |
| | | 0.33 | 8.9±0.1 | 11.±0.2 | 4.91±0.66 |
| | | 0.35 | 10.4±0.5 | 12.12±0.2 | 3.74±0.19 |
| 75/25 | 0.25 | 0.28 | - | - | * |
| | | 0.33 | - | - | * |
| | | 0.35 | - | - | * |
| | 0.50 | 0.28 | - | - | * |
| | | 0.33 | - | - | * |
| | | 0.35 | - | - | * |
| | 0.60 | 0.28 | - | - | * |
| | | 0.33 | - | - | * |
| | | 0.35 | - | - | * |
| | 0.75 | 0.28 | - | - | * |
| | | 0.33 | - | - | * |
| | | 0.35 | - | - | * |
| 65/35 | 0.25 | 0.28 | - | - | * |
| | | 0.33 | - | - | * |
| | | 0.35 | - | - | * |
| | 0.50 | 0.28 | - | - | * |
| | | 0.33 | - | - | * |
| | | 0.35 | - | - | * |
| | 0.60 | 0.28 | - | - | * |
| | | 0.33 | - | - | * |
| | | 0.35 | - | - | * |
| | 0.75 | 0.28 | - | - | * |
| | | 0.33 | - | - | * |
| | | 0.35 | - | - | * |

Table 2 shows that NH₄H₂PO₄ setting solution used in Control 2 cannot improve the (NH₄)₂HPO₄ setting solution used in Control 1 in term of the working/setting times and the 1 d-CS. Apparently the powder component having only TTCP and CSH phases dose not give a satisfactory result.

### Control 3: DCPA/CSH cement and (NH₄)₂HPO₄ setting solution

**Table 3. DCPA/CSH mixed with 0.25-0.75M (NH₄)₂HPO₄**

| DCPA/CSH (weight ratio) | (NH₄)₂HPO₄ Concentration (M) | L/P Ratio (cc/g) ml/g | WT (min) | ST (min) | 1 d-CS (MPa) |
|---|---|---|---|---|---|
| 90/10 | 0.25 | 0.28 | - | - | * |
| | | 0.33 | - | - | * |
| | | 0.35 | - | - | * |
| | 0.50 | 0.28 | - | - | □ |
| | | 0.33 | - | - | □ |
| | | 0.35 | - | - | □ |
| | 0.60 | 0.28 | - | - | □ |
| | | 0.33 | - | - | □ |
| | | 0.35 | - | - | □ |
| | 0.75 | 0.28 | - | - | □ |
| | | 0.33 | - | - | □ |
| | | 0.35 | - | - | □ |
| 75/25 | 0.25 | 0.28 | - | - | □ |
| | | 0.33 | - | - | □ |
| | | 0.35 | - | - | □ |
| | 0.50 | 0.28 | - | - | □ |
| | | 0.33 | - | - | □ |
| | | 0.35 | - | - | □ |
| | 0.60 | 0.28 | - | - | □ |
| | | 0.33 | - | - | □ |
| | | 0.35 | - | - | □ |
| | 0.75 | 0.28 | - | - | □ |
| | | 0.33 | - | - | □ |
| | | 0.35 | - | - | □ |
| 65/35 | 0.25 | 0.28 | - | - | * |
| | | 0.33 | - | - | * |
| | | 0.35 | - | - | * |
| | 0.50 | 0.28 | - | - | □ |
| | | 0.33 | - | - | □ |
| | | 0.35 | - | - | □ |
| | 0.60 | 0.28 | - | - | □ |
| | | 0.33 | - | - | □ |
| | | 0.35 | - | - | □ |
| | 0.75 | 0.28 | - | - | □ |
| | | 0.33 | - | - | □ |
| | | 0.35 | - | - | □ |

The molded lumps prepared from DCPA/CSH cement collapse and disintegrate when they are immersed in Hanks' solution (*), or their compressive strength cannot be measured after being immersed in Hanks' solution for 1 day (□) as shown in Table 3. Apparently the powder component having only DCPA and CSH phases does not give a satisfactory result.

### Control 4: DCPA/CSH cement and NH₄H₂PO₄ setting solution

**Table 4. DCPA/CSH mixed with 0.25-0.75M NH₄H₂PO₄**

| DCPA/CSH (weight ratio) | NH₄H₂PO₄ concentration (M) | L/P ratio (cc/g) ml/g | WT (min) | ST (min) | 1 d-CS (MPa) |
|---|---|---|---|---|---|
| 90/10 | 0.25 | 0.28 | - | - | □ |
| | | 0.33 | - | - | □ |
| | | 0.35 | - | - | □ |
| | 0.50 | 0.28 | - | - | □ |
| | | 0.33 | - | - | □ |
| | | 0.35 | - | - | □ |
| | 0.60 | 0.28 | - | - | □ |
| | | 0.33 | - | - | □ |
| | | 0.35 | - | - | □ |
| | 0.75 | 0.28 | - | - | □ |
| | | 0.33 | - | - | □ |
| | | 0.35 | - | - | □ |
| 75/25 | 0.25 | 0.28 | - | - | □ |
| | | 0.33 | - | - | □ |
| | | 0.35 | - | - | □ |
| | 0.50 | 0.28 | - | - | □ |
| | | 0.33 | - | - | □ |
| | | 0.35 | - | - | □ |
| | 0.60 | 0.28 | - | - | □ |
| | | 0.33 | - | - | □ |
| | | 0.35 | - | - | □ |
| | 0.75 | 0.28 | - | - | □ |
| | | 0.33 | - | - | □ |
| | | 0.35 | - | - | □ |
| 65/35 | 0.25 | 0.28 | - | - | □ |
| | | 0.33 | - | - | □ |
| | | 0.35 | - | - | □ |
| | 0.50 | 0.28 | - | - | □ |
| | | 0.33 | - | - | □ |
| | | 0.35 | - | - | □ |
| | 0.60 | 0.28 | - | - | □ |
| | | 0.33 | - | - | □ |
| | | 0.35 | - | - | □ |
| | 0.75 | 0.28 | - | - | □ |
| | | 0.33 | - | - | □ |
| | | 0.35 | - | - | □ |

The compressive strength of the molded lumps prepared from DCPA/CSH cement cannot be measured after being immersed in Hanks' solution for 1 day (□) as shown in Table 4. Apparently the powder component having only DCPA and CSH phases does not give a satisfactory result.

### For Tables 5-14

| TTCP/DCPA : CSH (weight ratio) | TTCP : DCPA : CSH (weight ratio) |
|---|---|
| 90 : 10 | 2.69 : 1 : 0.41 |
| 85 : 15 | 2.69 : 1 : 0.65 |
| 80 : 20 | 2.69 : 1 : 0.92 |
| 75 : 25 | 2.69 : 1 : 1.23 |
| 65 : 35 | 2.69 : 1 : 1.99 |
| 55 : 45 | 2.69 : 1 : 3.02 |
| 45 : 55 | 2.69 : 1 : 4.51 |
| 35 : 65 | 2.69 : 1 : 6.85 |
| 25 : 75 | 2.69 : 1 : 11.07 |
| 10 : 90 | 2.69 : 1 : 33.21 |

| | |
|---|---|
| Note: TTCP and DCPA have a molar ratio of 1:1 under all conditions | |

### Example 1: (TTCP/DCPA)/CSH cement and various setting solutions

**Table 5. 75 wt% phosphate (TTCP/DCPA)/25 wt% CSH composite cement**

| Setting solution | Conc. (M) | Solution pH value | WT (min) | ST (min) | 1 d-CS (MPa) |
|---|---|---|---|---|---|
| K₂HPO₄ | 1 | 9.27 | 1.53±0.21 | 2.72±0.26 | 15.35±0.85 |
| (L/P=0.33) | 0.75 | 9.20 | 1.88±0.13 | 3.08±0.15 | 15.46±3.51 |
| | 0.5 | 9.17 | 2.85±0.13 | 3.93±0.16 | 18.39±1.53 |
| | 0.25 | 9.15 | -- | -- | 24.12±6.30 |
| (NH₄)₂HPO₄ | 1 | 8.17 | 4.88±0.13 | 6.93±0.25 | 27.77±1.6 |
| (L/P=0.33) | 0.75 | 8.09 | 5.47±0.13 | 7.45±0.16 | 25.37±0.69 |
| | 0.6 | 7.97 | 5.77±0.13 | 7.53±0.27 | 41.02±3.77 |
| | 0.5 | 7.95 | 7.85±0.32 | 10.05±0.33 | 21.58±1.82 |
| | 0.25 | 7.92 | 13.5±0.5 | 16.28±1.1 | * |
| (NH₄)H₂PO₄ | 1 | 3.96 | 4.95±0.5 | 7.5±0.5 | * |
| (L/P=0.33) | 0.75 | 3.99 | 6.63±0.38 | 8.93±0.33 | * |
| | 0.5 | 4.28 | 9.43±0.416 | 12.6±0.53 | * |
| | 0.25 | 4.30 | 11.42±0.5 | 15.18±0.27 | * |
| NaH₂PO₄·2H₂O | 1 | 3.89 | 6.5±0.43 | 9±0.33 | 23.82±3.23 |
| (L/P=0.33) | 0.75 | 3.97 | 8.05±0.25 | 11.05±0.75 | 18.96±1.79 |
| | 0.5 | 4.13 | 10.72±0.25 | 14.68±0.28 | * |
| | 0.25 | 4.22 | 12.92±0.67 | 16.16±1.03 | * |

It can be summarized from the data shown in Table 5 as follows:
1. K₂HPO₄-derived hardened cement composites have too short WT/ST and low CS.
2. ((NH₄) H₂PO4-derived hardened cement composites have reasonable WT/ST, but is dispersed after immersion in Hanks' solution.
3. NaH₂PO₄·2H₂O-derived hardened cement composites have reasonable WT/ST, but too acidic and low strength.
4. Among all setting solutions tested, (NH₄)₂HPO₄ results in the highest CS.
5. Among all (NH₄)₂HPO₄ concentrations, 0.6 M gives the highest CS (41 MPa).

### Example 2: TTCP/DCPA/CSH mixed with 0.60M (NH₄)₂HPO₄

**Table 6. TTCP/DCPA/CSH mixed with 0.60 M (NH₄)₂HPO₄.**

| TTCP/DCPA : CSH (weight ratio) | Setting solution | L/P ratio (cc/g) | 1 d-CS (MPa) | WT/ST (Min) | Cytotoxicity O.D. (%) (Al₂O₃ as control-100%) |
|---|---|---|---|---|---|
| 90 : 10 | 0.60 M (NH₄)₂HPO₄ | 0.28 | 45.10±3.50 | 6.1±0.1/ 7.1±0.1 | 86.40±2.20 |
| 85 : 15 | 0.60 M (NH₄)₂HPO₄ | 0.28 | 44.50±5.50 | 5.8±0.1/ 6.9±0.2 | 91.76±4.90 |
| 80 : 20 | 0.60 M (NH₄)₂HPO₄ | 0.28 | 40.93±3.98 | 6.6±0.3/ 7.7±0.3 | 89.32±0.28 |
| 75 : 25 | 0.60 M (NH₄)₂HPO₄ | 0.28 | 40.45±2.39 | 5.8±0.1/ 7.5±0.3 | 95.49±3.04 |
| 65 : 35 | 0.60 M (NH₄)₂HPO₄ | 0.28 | 26.53±1.57 | 5.5±0.3/ 7.5±0.4 | 84.94±2.98 |
| 55 : 45 | 0.60 M (NH₄)₂HPO₄ | 0.28 | 28.07±1.54 | 5.2±0.2/ 6.8±0.3 | 101.99±0.74 |
| 45 : 55 | 0.60 M (NH₄)₂HPO₄ | 0.28 | 27.02±1.29 | 5.5±0.2/ 6.9±0.1 | 89.29±10.44 |
| 35 : 65 | 0.60 M (NH₄)₂HPO₄ | 0.28 | 14.10±3.10 | 4.4±0.3/ 6.4±0.4 | 97.31±1.91 |
| 25 : 75 | 0.60 M (NH₄)₂HPO₄ | 0.28 | 13.80±1.61 | 5.1±0.1/ 6.1±0.4 | 85.97±3.94 |
| 10 : 90 | 0.60 M (NH₄)₂HPO₄ | 0.28 | 9.77±1.15 | 5.0±0.2/ 7.9±0.2 | 93.17±8.63 |

Summary of the results shown in Table 6 (TTCP/DCPA/CSH mixed with 0.60 M (NH₄)₂HPO₄):
(1)When CSH contents are higher than about 65 wt% in the TTCP/DCPA/CSH cement powder, their CS values become too low (< 15 MPa). The appropriate CSH content under the test conditions should be less than about 65 wt%. To obtain a higher strength (CS > 30 MPa), the CSH content should be less than about 35 wt%.
(2) All cytotoxicity values are acceptably greater than 80% for all CSH contents (from about 10 wt% to about 90 wt%) when 0.60 M (NH₄)₂HPO₄ is used as hardening solution.

### Example 3: TTCP/DCPA/CSH mixed with 0.20-3.0 M (NH₄)₂HPO₄

**Table 7. TTCP/DCPA/CSH (TTCP/DCPA : CSH = 90:10) mixed with 0.20-3.00M (NH₄)₂HPO₄**

| Powder | (NH₄)₂HPO₄ concentration (M) | L/P ratio (cc/g) mL/g | 1d-CS (MPa) | WT/ST (Min) | Cytotoxicity O.D. (%) (Al₂O₃ as control-100%) |
|---|---|---|---|---|---|
| TTCP/DCPA : CSH (90:10) | 0.20 | 0.28 | * | - | |
| | 0.25 | 0.28 | * | 8.1±0.2/ 9.9±0.2 | 104.95±6.46 |
| | 0.50 | 0.28 | 32.37±3.10 | 5.9±0.3/ 7.2±0.7 | 91.31±5.77 |
| | 0.60 | 0.28 | 45.11±3.50 | 6.1±0.2/ 7.1±0.1 | 91.61±6.06 |
| | 0.75 | 0.28 | 35.48±1.8 | 5.9±0.3/ 6.9±0.2 | 86.99±5.13 |
| | 1.00 | 0.28 | 35.79±2.60 | 5.7±0.1/ 6.3±0.1 | 74.09±3.44 |
| | 2.00 | 0.28 | 41.75±2.60 | 4.5±0.5/ 5.9±0.3 | 57.85±4.22 |
| | 3.00 | 0.28 | 42.68±2.60 | 4.7±0.2/ 6.0±0.1 | 46.80±5.04 |

Summary of the results shown in Table 7 (TTCP/DCPA/CSH (TTCP/DCPA : CSH = 90:10) mixed with 0.20-3.00M (NH₄)₂HPO₄):
(1) When the (NH₄)₂HPO₄ concentration is too low (0.25 M or lower under the present test conditions), the hardened paste is dispersed upon immersion in Hanks' solution and its strength cannot be measured.
(2) Although ammonium is a critical life-sustaining element, the (NH₄)₂HPO₄ concentration cannot be too high (2 M or higher under the present test conditions) for reducing cytotoxicity level.
(3) The appropriate (NH₄)₂HPO₄ concentration should be in the window of 0.25 M-2.0 M, preferably 0.5 M-1.0 M. (or the NH₄⁺ ion concentration in the window of 0.50 M-4.0 M, preferably 1.0 M-2.0 M)

### Example 4: TTCP/DCPA/CSH mixed with 0.20-3.0 M (NH₄)₂HPO₄

**Table 8. TTCP/DCPA/CSH (TTCP/DCPA : CSH = 75:25) mixed with 0.20-3.00M (NH₄)₂HPO₄**

| Powder | (NH₄)₂HPO₄ concentration (M) | UP Ratio (cc/g) | 1d-CS (MPa) | WT/ST (Min) | Cytotoxicity O.D.(%) (Al₂O₃ as control-100%) |
|---|---|---|---|---|---|
| TTCP/DCPA: CSH (75:25) | 0.20 | 0.28 | * | - | - |
| | 0.25 | 0.28 | * | 13.5±0.5/ 16.3±1.1 | 98.94±3.41 |
| | 0.50 | 0.28 | 21.58±1.82 | 7.9±0.3/ 10.1±0.3 | 90.83±6.02 |
| | 0.60 | 0.28 | 40.45±2.39 | 5,8±0.1/ 7.5±0.3 | 88.17±1.39 |
| | 0.75 | 0.28 | 25.37±0.69 | 5.5±0.1/ 7.5±0.2 | 93.32±5.01 |
| | 1.00 | 0.28 | 27.77±1.60 | 4.9±0.1/ 6.9±0.3 | 91.21±1.99 |
| | 2.00 | 0.28 | 24.53±1.80 | 3.6±0.5/ 4.8±0.3 | 74.42±3.25 |
| | 3.00 | 0.28 | 32.86±2.10 | 2.9±0.1/ 4.2±0.3 | 59.79±3.23 |

Summary of the results shown in Table 8 (TTCP/DCPA/CSH (TTCP/DCPA : CSH = 75:25) mixed with 0.20-3.00M (NH₄)₂HPO₄):
(1)When the (NH₄)₂HPO₄ concentration is too low (0.25 M or lower under the present test conditions), the hardened paste is dispersed upon immersion in Hanks' solution and its strength cannot be measured.
(2)Although ammonium is a critical life-sustaining element, the (NH₄)₂HPO₄ concentration cannot be too high (> 2 M under the present test conditions) for reducing cytotoxicity level.
(3) The appropriate (NH₄)₂HPO₄ concentration should be in the window of 0.25 M-2.0 M, preferably 0.5 M-1.0 M. (or the NH₄⁺ ion concentration in the window of 0.50 M-4.0 M, preferably 1.0 M-2.0 M)

### Example 5: TTCP/DCPA/CSH mixed with 0.25-1.0 M (NH₄)₂HPO₄

**Table 9. TTCP/DCPA/CSH (TTCP/DCPA : CSH = 65:35) mixed with 0.25-1.0M (NH₄)₂HPO₄**

| Powder | (NH₄)₂HPO₄ concentration (M) | L/P (cc/g) ml/g | 1-d CS (MPa) |
|---|---|---|---|
| TTCP/DCPA: CSH (65:35) | 1.00 | 0.35 | 21.24±1.82 |
| | | 0.33 | 23.12±1.21 |
| | | 0.30 | 23.33±1.38 |
| | | 0.28 | 23.52±1.67 |
| | 0.75 | 0.35 | 24.32±1.56 |
| | | 0.33 | 25.62±0.93 |
| | | 0.30 | 24.36±1.62 |
| | | 0.28 | 29.54±1.13 |
| | 0.50 | 0.35 | 18.36±1.45 |
| | | 0.33 | 22.59±1.61 |
| | | 0.30 | 26.75±1.51 |
| | | 0.28 | 31.37±0.81 |
| | 0.45 | 0.35 | 34.23±0.61 |
| | | 0.33 | 30.77±1.57 |
| | | 0.30 | 25.01±0.56 |
| | | 0.28 | 22.65±1.23 |
| | 0.40 | 0.35 | 27.32±1.45 |
| | | 0.33 | 32.51±0.72 |
| | | 0.30 | 28.77±1.22 |
| | | 0.28 | 14.16±1.15 |
| | 0.25 | 0.35 | * |
| | | 0.33 | * |
| | | 0.30 | * |
| | | 0.28 | * |

Summary of the results shown in Table 9 (TTCP/DCPA/CSH (TTCP/DCPA : CSH = 65:35) mixed with 0.25-1.0M (NH₄)₂HPO₄):
(1) When the (NH₄)₂HPO₄ concentration is too low (0.25M or lower under the present test conditions), the hardened paste is dispersed upon immersion in Hanks' solution and its strength cannot be measured.
(2) Except a few cases, all 1-d CS values are higher than 20 MPa, and, under certain conditions, higher than 30 MPa.

### Example 6: TTCP/DCPA/CSH mixed with 0.40-1.0 M (NH₄)₂HPO₄

**Table 10. TTCP/DCPA/CSH (TTCP/DCPA : CSH = 55:45) mixed with 0.40-1.00M (NH₄)₂HPO₄**

| Powder | (NH₄)₂HPO₄ concentration (M) | L/P Ratio ml/g (cc/g) | WT (min) | ST (min) | 1-d CS (MPa) |
|---|---|---|---|---|---|
| TTCP/DCPA : CSH (55:45) | 0.40 | 0.35 | 10.3±0.3 | 11.9±0.1 | 28.87±2.52 |
| | | 0.33 | 9.3±0.3 | 10.9±0.2 | 30.46±1.89 |
| | | 0.30 | 7.9±0.5 | 9.4±0.3 | 26.62±2.23 |
| | | 0.28 | 6.9±0.4 | 8.6±0.2 | 24.96±3.79 |
| | 0.45 | 0.35 | 8.9±0.1 | 10.8±0.2 | 27.93±2.69 |
| | | 0.33 | 7.7±0.3 | 9.6±0.2 | 26.22±2.51 |
| | | 0.30 | 6.6±0.2 | 8.5±0.3 | 26.71±1.41 |
| | | 0.28 | 5.4±0.4 | 7.4±0.4 | 27.82±2.59 |
| | 0.50 | 0.35 | 9.2±0.1 | 11.2±0.2 | 24.67±2.23 |
| | | 0.33 | 8.4±0.3 | 10.3±0.2 | 27.86±2.26 |
| | | 0.30 | 7.2±0.2 | 9.2±0.3 | 32.05±3.02 |
| | | 0.28 | 6.0±0.3 | 7.5±0.3 | 34.70±1.52 |
| | | 0.26 | 5.4±0.2 | 7.4±0.1 | 30.50±3.77 |
| | 0.60 | 0.35 | 8.5±0.3 | 10.3±0.3 | 28.60±1.99 |
| | | 0.33 | 7.3±0.3 | 9.2±0.2 | 27.10±1.28 |
| | | 0.3 | 6.3±0.1 | 8.4±0.1 | 25.74±2.20 |
| | | 0.28 | 5.2±0.2 | 6.8±0.3 | 28.07±1.54 |
| | 0.75 | 0.35 | 6.4±0.4 | 8.5±0.2 | 23.40±3.55 |
| | | 0.33 | 5.3±0.3 | 7.5±0.4 | 29.25±1.45 |
| | | 0.30 | 5.2±0.3 | 7.1±0.2 | 29.59±2.65 |
| | | 0.28 | 4.7±0.3 | 6.5±0.3 | 30.00±2.83 |
| | 1.00 | 0.35 | 5.3±0.1 | 7.0±0.2 | 28.46±3.38 |
| | | 0.33 | 4.5±0.2 | 6.2±0.2 | 30.71±2.76 |
| | | 0.30 | 3.7±0.3 | 5.3±0.1 | 25.71±3.86 |
| | | 0.28 | 2.9±0.3 | 4.2±0.3 | 29.58±1.24 |

Summary of the results shown in Table 10 (TTCP/DCPA/CSH (TTCP/DCPA : CSH = 55:45) mixed with 0.40-1.00M (NH₄)₂HPO₄):
(1) All 1-d CS values are higher than 20 MPa, and, under certain conditions, higher than 30 MPa.
(2) For higher concentration (1.0M) and lower L/P values (lower than 0.33 cc/g), the WT/ST are a little too short.

### Example 7: TTCP/DCPA/CSH mixed with 0.40-0.60 M (NH₄)₂HPO₄

**Table 11. TTCP/DCPA/CSH (TTCP/DCPA : CSH = 45:55) mixed with 0.40-0.60 M (NH₄)₂HPO₄**

| Powder | (NH₄)₂HPO₄ concentration (M) | L/P Ratio (cc/g) | WT (min) | ST (min) | 1-d CS (MPa) |
|---|---|---|---|---|---|
| TTCP/DCPA : CSH (45:55) | 0.40 | 0.35 | 8.2±0.3 | 10.7±0.2 | 21.1±1.0 |
| | | 0.33 | 7.5±0.2 | 9.6±0.3 | 23.3±2.0 |
| | | 0.30 | 6.1±0.3 | 8.3±0.2 | 27.1±2.2 |
| | 0.50 | 0.35 | 7.6±0.3 | 10.2±0.2 | 22.3±0.5 |
| | | 0.33 | 7.0±0.5 | 9.1±0.2 | 22.1±2.2 |
| | | 0.30 | 5.8±0.2 | 7.8±0.1 | 24.1±1.6 |
| | 0.60 | 0.35 | 7.9±0.3 | 10.0±0.2 | 21.4±2.0 |
| | | 0.33 | 6.6±0.4 | 9.0±0.6 | 23.8±1.7 |
| | | 0.30 | 5.5±0.1 | 7.5±0.2 | 23.8±1.7 |

Summary of the results shown in Table 11 (TTCP/DCPA/CSH (TTCP/DCPA : CSH = 45:55) mixed with 0.40-0.60M (NH₄)₂HPO₄):
(1) All 1-d CS values are higher than 20 MPa
(2) Working times are longer than 5.5 min and setting times are longer than 7.5 min under all testing conditions.

**Table 12. CS (MPa) of TTCP/DCPA/CSH after immersion in Hanks' solution for different days**

| TTCP/DCPA : CSH (weight ratio) | Setting solution | L/P ratio (cc/g) ml/g | 1d-CS | 3d-CS | 7d-CS | 14d-CS | 28d-CS | 42d-CS |
|---|---|---|---|---|---|---|---|---|
| 90:10 | 0.60M (NH₄)₂HPO₄ | 0.28 | 44.60±3.64 | 40.52±4.68 | 40.17±3.47 | 39-86±4.96 | 39.46±3.17 | 37.98±3.79 |
| 85:15 | 0.60M (NH₄)₂HPO₄ | 0.28 | 43.70±4.71 | 41.56±4.23 | 40.20±3.62 | 38.47±3.32 | 37.44±4.68 | 35.24±5.13 |
| 80:20 | 0.60M (NH₄)₂HPO₄ | 0.28 | 40.17±4.76 | 37.13±5.14 | 36.05±3.47 | 35.76±4.79 | 35.26±3.46 | 34.77±3.79 |
| 75:25 | 0.60M (NH₄)₂HPO₄ | 0.28 | 40.45±2.39 | 39.25±3.25 | 38.43±1.87 | 38.34±2.15 | 36.25±1.51 | 32.54±2.39 |
| 65:35 | 0.45M (NH₄)₂HPO₄ | 0.35 | 34.23±0.60 | 33.65±1.75 | 31.25±1.87 | 30.82±2.15 | 29,47±1.66 | 27.76±2.59 |
| 55:45 | 0.60M (NH₄)₂HPO₄ | 0.35 | 28.60±2.00 | 19.58±1.40 | 20.02±1.77 | 14.59±1.72 | 11.67±1.22 | 8.09±1.04 |

Summary of the results shown in Table 12:
(1) Except the "55:45" composite, all 42d-CS values are higher than 20 MPa, indicating a mild decay in strength even after immersion in Hanks' solution for 42 days. W hen the CSH content is increased to 45 wt%, however, the CS value of the composite decreases very significantly.

### Example 8: Effects of addition of organic acids on TTCP/DCPA/CSH mixed with 1.0 M (NH₄)₂HPO₄

**Table 13. Effects of addition of organic acids (tartaric acid, citric acid and malic acid) on properties of TTCP/DCPA/CSH (TTCP/DCPA : CSH = 75:25) mixed with 1 M (NH₄)₂HPO₄**

| Organic acid | Concentration of organic acid (M) | L/P Ratio ml/g (cc/g) | Solution pH | WT (min) | ST (min) | 1 d-CS (MPa) |
|---|---|---|---|---|---|---|
| Malic acid | 0.4 | 0.40 | 5.31 | 7.8 ±0.2 | 9.9 ±0.4 | 20.17±2.20 |
| | 0.3 | 0.40 | 5.86 | 8.4 ±0.4 | 10.5±0.5 | 24.15±2.73 |
| | 0.2 | 0.40 | 6.39 | 9.0±0.5 | 10.50±0.5 | 23.44±3.79 |
| | 0.1 | 0.40 | 6.98 | 9.4 ±0.4 | 10.4 ±0.5 | 23.90±3.05 |
| Citric acid | 0.4 | 0.33 | 4.71 | 2.4±0.3 | 4.0±0.3 | 27.65±3.24 |
| | 0.3 | 0.33 | 5.28 | 4.7±0.6 | 7.1 ±0.1 | 30.08±1.44 |
| | 0.2 | 0.33 | 5.94 | 7.0±0.3 | 10.0±0.4 | 29..48±1.46 |
| | 0.1 | 0.33 | 6.71 | 9.2±0.4 | 10.3 ±0.4 | 31.66±2.49 |
| Tartaric acid | 0.4 | 0.33 | 5.10 | 3.9±0.3 | 6.8±0.2 | 32.38±2.48 |
| | 0.35 | 0.33 | 5.57 | 4.2±0.2 | 7.1±0.1 | 34.60±3.70 |
| | 0.3 | 0.33 | 5.62 | 6.0±0.5 | 9.8 ±0.2 | 30.97±2.86 |
| | 0.2 | 0.33 | 6.25 | 7.2±0.4 | 10.2 ±0.4 | 26.04±3.64 |
| | 0.1 | 0.33 | 6.99 | 9.4±0.4 | 11.2±0.2 | 27.36±5.31 |

Summary of the results shown in Table 13:
(1) Addition of malic acid in 1 M (NH₄)₂HPO₄ setting solution decreases the CS value of the hardened composite cement
(2) Addition of citric acid in 1 M (NH₄)₂HPO₄ setting solution increases the CS value of the hardened composite cement (from about 28 MPa to about 32 MPa)
(3) Addition of tartaric acid in 1 M (NH₄)₂HPO₄ setting solution increases the CS value of the hardened composite cement (from about 28 MPa to 35 MPa)

### Preparation of a TTCP/DCPA/CSH composite dense block

Appropriate amounts of TTCP and DCPA powders were uniformly mixed in a ball miller, followed by uniformly mixing with appropriate amount of CSH powder. The resultant TTCP/DCPA/CSH mixed powders were mixed uniformly with a desirable setting solution (e.g., 0.6M (NH₄)₂HPO₄) at a desirable L/P ratio (e.g., 0.28 cc/g) to form a TTCP/DCPA/CSH paste.

Prior to being fully hardened, the paste was placed in a mold under a desirable pressure (at a maximum pressure of 1.47, 2.94 or 4.41 KN (150, 300 or 450 Kgf) to squeeze a portion of the liquid out of the paste. After being removed from the mold, one group of the hardened composite samples were placed in a moisture-proof container for 1 day. Another group of samples were further impregnated in an impregnation solution (1M (NH₄)₂HPO₄ or 1M K₂HPO₄ at a desirable temperature (37°C) for 1 day, followed by drying in an oven at 50°C for 1 day.

### Preparation of TTCP/DCPA/CSH composite porous blocks

Appropriate amounts of TTCP and DCPA powders were uniformly mixed in a ball miller, followed by uniformly mixing with appropriate amount of CSH powder. The resultant TTCP/DCPA/CSH mixed powders were mixed uniformly with a desirable setting solution (e.g., 0.6M (NH₄)₂HPO₄) at a desirable L/P ratio (e.g., 0.28 cc/g) to form a TTCP/DCPA/CSH paste.

The composite paste was then uniformly mixed with a pore-forming agent (e.g. KCl particles) with a desirable weight ratio (e.g. TTCP/DCPA/CSH : KCl = 1:1 or 1:2) to form a TTCP/DCPA/CSH/KCl paste.

Prior to being fully hardened, the composite paste was placed in a mold under a desirable pressure (at a maximum pressure of 4.41 KN (450 Kgf) to squeeze a portion of the liquid out of the paste. After being removed from the mold, one group of the hardened composite blocks were immersed in de-ionized water at 37°C for 3 days to allow KCl particles to dissolve, forming a porous composite block, followed by drying in an oven at 50°C for 1 day. Another group of samples were further impregnated in an impregnation solution (e.g. 1M (NH₄)₂HPO₄ or 1M K₂HPO₄ at a desirable temperature (37°C) for 1 day to allow the strength of the porous block to increase, followed by drying in an oven at 50°C for 1 day. To remove the residual impregnation solution from inside the pores, the impregnated porous samples were further rinsed in de-ionized water at 37°C for 3 days.

### Measurement of porosity

The porosity of the various samples was measured according to ASTM C830-00 (2006) method, "Standard Test Methods for Apparent Porosity, Liquid Absorption, Apparent Specific Gravity, and Bulk Density of Refractory Shapes by Vacuum Pressure".

### Example 9: Dense block

**Table 14. Compressive strengths (MPa) of TTCP/DCPA/CSH composite dense blocks prepared from TTCP/DCPA/CSH mixed powders mixed with 0.6 M (NH₄)₂HPO₄ (L/P=0.28 mL/g (cc/g))**

| TTCP/DCPA: CSH (weight ratio) | Without impregnation treatment | | | Impregnated in 1 M (NH₄)₂HPO₄, at 37°C for 1d | | | Impregnated in 1M K₂HPO₄, at 37°C for 1d | | |
|---|---|---|---|---|---|---|---|---|---|
| | Molding pressure (kgf) | | | Molding pressure (kgf) | | | Molding pressure (kgf) | | |
| | (150) 1.47 kN | (300) 2.94 kN | (450) 4.41 kN | (150) 1.47 kN | (300) 2.94 kN | (450) 4.41 kN | (150) 1.47 kN | (300) 2.94 kN | (450) 4.41 kN |
| 90:10 | 42.74 ±3.20 | 49.21 ±3.17 | 55.86 ±1.67 | 101.38 ±9.23 | 130.83 ±6.88 | 167.02 ±7.57 | 111.07 ±6.93 | 162.96 ±8.07 | 160.07 ±4.13 |
| 85:15 | 38.11 ±2.51 | 49.21 ±3.69 | 53.55 ±1.14 | 100.26 ±7.06 | 127.49 ±11.21 | 162.95 ±6.26 | 105.78 ±6.81 | 142.01 ±8.92 | 150.07 ±3.25 |
| 75:25 | 38.02 ±3.00 | 46.34 ±1.79 | 54.78 ±7.45 | 103.07 ±6.55 | 121.68 ±5.07 | 137.99 ±9.58 | 105.32 ±7.02 | 129.10 ±9.81 | 144.90 ±11.33 |
| 65:35 | 36.14 ±1.64 | 46.32 ±8.16 | 60.64 ±2.34 | 101.04 ±4.77 | 120.53 ±7.98 | 134.78 ±9.74 | 98.26 ±10.71 | 131.78 ±6.77 | 136.85 ±8.47 |
| 55:45 | 35.59 ±1.32 | 48.71 ±2.80 | 54.95 ±4.70 | 95.14 ±6.06 | 121.91 ±7.68 | 128.63 ±6.92 | 90.18 ±3.63 | 132.57 ±5.28 | 140.37 ±3.45 |

Summary of the results shown in Table 14 (Compressive strengths of TTCP/DCPA/CSH composite dense blocks):
(1) Impregnation-treated samples have significantly higher CS values under all conditions
(2) CS values significantly increase with higher molding pressure under all conditions

### Example 10: Porous block

**Table 15. Porosity values (vol%) of TTCP/DCPA/CSH composite porous blocks prepared from TTCP/DCPA/CSH/KCl (pore-forming agent)-mixed powders mixed with 0.6 M (NH₄)₂HPO₄ (L/P=0.33 mL/g (cc/g))**

| TTCP/DCPA: CSH (weight ratio) | TTCP/DCPA/CSH : KCl = 1:1 (weight ratio) | TTCP/DCPA/CSH : KCl = 1:2 (weight ratio) |
|---|---|---|
| 90:10 | 46.02 ± 2.87 | 81.51 ± 6.57 |
| 85:15 | 57.66 ± 0.98 | 83.77 ± 5.61 |
| 75:25 | 59.82 ± 1.16 | 84.32 ± 6.71 |
| 65:35 | 63.02 ± 1.41 | 87.86 ± 3.74 |
| 55:45 | 59.98 ± 0.87 | 88.69 ± 5.72 |

Summary of the results shown in Table 15 (TTCP/DCPA/CSH composite porous blocks prepared from TTCP/DCPA/CSH/KCI (pore-forming agent)-mixed powders mixed with 0.6 M (NH₄)₂HPO₄):
(1) The porosity values are in the range 46-63% at TTCP/DCPA/CSH : KCl = 1:1, and 81-89% at TTCP/DCPA/CSH : KCl = 1:2, which are ideal for use as a tissue-engineered scaffold.
(2) The porosity value generally increases with increasing CSH content in the composite.

**Table 16. Compressive strength (MPa) values of TTCP/DCPA/CSH composite porous blocks prepared from TTCP/DCPA/CSH/KCI (pore-forming agent)-mixed powders mixed with 0.6 M (NH₄)₂HPO₄ (L/P=0.33 ml/g (cc/g))**

| TTCP/DCPA : CSH (weight ratio) | Without impregnation TTCP/DCPA/CSH : KCI = 1:1 (weight ratio) | Without impregnation TTCP/DCPA/CSH : KCI= 1:2 (weight ratio) | Impregnated in 1M(NH₄)₂HPO₄, at 37°C for 1 d TTCP/DCPA/CSH KCI = 1:1 (weight ratio) | Impregnated in 1M (NH₄)₂HPO₄, at 37°C for 1 d TTCP/DCPA/CSH : KCI = 1:2 (weight ratio) | Impregnated in 1M K₂HPO₄, at 37°C for 1 d TTCP/DCPA/CSH : KCI = 1:1 (weight ratio) | Impregnated in 1M K₂HPO₄, at 37°C for 1 d TTCP/DCPA/CSH : KCI = 1:2 (weight ratio) |
|---|---|---|---|---|---|---|
| 90:10 | 4.86±0.43 | 0.92±0.18 | 8.43±0.73 | 1.62±0.28 | 8.71±0.93 | 1.50±0.30 |
| 85:15 | 5.00±0.64 | 0.82±0.23 | 7.29±0.54 | 1.22±0.19 | 7.37±0.98 | 1.36±0.19 |
| 75:25 | 4.18±0.49 | 0.74±0.10 | 7.03±0.49 | 0.98±0.18 | 7.40±0.73 | 0.93±0.23 |
| 65:35 | 3.74±0.42 | 0.51 ±0.07 | 5.19±0.26 | 0.91±0.05 | 5.15±0.70 | 0.87±0.09 |
| 55:45 | 2.36±0.19 | 0.42 ±0.06 | 4.01±0.56 | 0.71±0.10 | 4.55±0.33 | 0.64±0.07 |

Summary of the results shown in Table 16 (TTCP/DCPA/CSH composite porous blocks prepared from TTCP/DCPA/CSH/KCI (pore-forming agent)-mixed powders mixed with 0.6M (NH₄)₂HPO₄):
(1) The CS strength of the porous blocks decreases with increasing CSH content in the composite under all test conditions.
(2) The CS values of the porous blocks (w/o impregnation treatment) prepared from TTCP/DCPA/CSH/KCl are in the range about 2-5 MPa at TTCP/DCPA/CSH : KCl=1:1, and about 0.4-0.9 MPa at TTCP/DCPA/CSH : KCl=1:2.
(3) The impregnation treatment significantly enhances the strength of the porous blocks. The CS values of (NH₄)₂HPO₄-impregnated porous blocks significantly increase to 4.8-8.9 MPa at TTCP/DCPA/CSH : KCl = 1:1, and about 0.5-1.2 MPa at TTCP/DCPA/CSH : KCl = 1:2. The CS values of K₂HPO₄-impregnated porous blocks significantly increase to 4.8-8.8 MPa at TTCP/DCPA/CSH : KCI = 1:1, and about 0.5-1.2 MPa at TTCP/DCPA/CSH : KCl = 1:2.

### Example 11: Animal study and measurement of composite implant resorption ratios

Animal study was performed at National Cheng-Kung University Medical College Animal Center, Tainan, Taiwan. Adult (weighing 2.8-3.5 kg), healthy, male New Zealand white rabbits were used as experimental animals. The rabbits were housed individually in stainless steel cages which had free access to food and water. An acclimation period of a minimum of 7 days was allowed between receipt of the animals and the start of the study. Injection sites were shaved and cleansed with 70% ethanol and Betadine™ (povidone iodine 10%). All animals were operated under general anesthesia. Pentobarbital sodium (0.1 ml/100 g, Tokyo Kasei Kogyo, Tokyo, Japan) was used as general anesthesia, while xylocaine (Fujisawa Pharmaceutical CO., Tokyo, Japan) was used as local anesthesia. To implant cement paste in the medial condyle of femur, a longitudinal incision was made on the anterior surface of the femur. The inner side of knee joint of the rabbit was cut to expose the femur. After exposure of the femur, the periosteum was reflected and a 2 mm pilot hole was drilled. The hole was gradually widened with drills of increasing size until a final diameter of 5 mm was reached. A special 5 mm diameter drill burr was used and a ring was inserted at a depth of 10mm to ensure appropriate length (10 mm) of the drill hole.

Two kinds of calcium phosphate/calcium sulfate composite cement paste (90wt% TTCP/DCPA: 10wt% CSH and 55wt% TTCP/DCPA: 45wt% CSH) were implanted in the prepared bone cavity. After filling of the paste, subcutaneous tissues and skin were closed up layer by layer with silk threads. To reduce the risk of peri-operative infection, the rabbits were treated with antibiotics injection subcutaneously at a dose of 40 mg/kg. The animals were sacrificed after 12 week post-operation.

After the animals were sacrificed, the femur portions were excised immediately and excess tissue was removed. Photographs of the slices by a single-lens reflex camera and an image analysis system was used to calculate the areas of residual implant. The implant resorption ratios were determined by the equation, implant resorption ratio = (cross-sectional area of original implant - cross-sectional area of residual implant)/cross-sectional area of original implant.

### Summary:

The average residual implant ratios for "90/10" and "55/45" samples are 81.1% (resorption ratio: 18.9%) and 67.7% (resorption ratio: 32.3%), respectively, as shown in the photographs mentioned above. That means the healing speed for the 55/45 implant is about 70% faster than for the 90/10 implant.

## Claims

1. A bone cement formula comprising a powder component and a setting liquid component with a liquid to powder ratio of 0.20 ml/g (cc/g) to 0.50 ml/g (cc/g), preferably 0.25 ml/g (cc/g) to 0.35 ml/g (cc/g),
wherein the powder component comprises a calcium sulfate source and a calcium phosphate source with a weight ratio of the calcium sulfate source greater than 5% and not greater than 55%, based on the total weight of the calcium sulfate source and the calcium phosphate source,
wherein the calcium sulfate source is calcium sulfate hemihydrate (CSH), calcium sulfate dehydrate (CSD), or anhydrous calcium sulfate, and preferably, CSH, and the calcium phosphate source comprises tetracalcium phosphate (TTCP) and dicalcium phosphate in a molar ratio of TTCP to dicalcium phosphate of 0.5 to 2.5, preferably 1.0, and
wherein the setting liquid component comprises ammonium ion (NH₄⁺) in a concentration of 0.5 M to 4 M.

2. The formula of claim 1, wherein the dicalcium phosphate is dicalcium phosphate anhydrous (DCPA).

3. The formula of claim 1, wherein the setting liquid component comprises ammonium ion (NH₄⁺) in a concentration of 1.0 M to 2.0 M.

4. The formula of claim 3, wherein the setting liquid component is a solution of NH₄H₂PO₄, (NH₄)₂HPO₄, (NH₄)₃PO₄·3H₂O or a mixture of them.

5. The formula of claim 4, wherein the setting liquid component is an aqueous solution of (NH₄)₂HPO₄.

6. The formula of claim 2, wherein the calcium phosphate source is composed of TTCP and DCPA.

7. The formula of claim 1 further comprising a pore-forming agent which is to be dissolved in a solution when a hardened bone cement composite prepared therefrom is immersed in the solution.

8. A process for preparing a hardened bone cement composite comprising forming a bone cement paste by mixing the powder component and the setting liquid component of the bone cement formula set forth in any one of claims 1 to 6; shaping the paste in a mold; and removing the mold to form a block of hardened bone cement composite.

9. The process of claim 8 further comprising pressurizing said paste in said mold before said paste becomes set to remove a portion of liquid from said paste, so that a liquid to powder ratio of said paste decreases, wherein the pressure applied to the paste in the mold is form 1 MPa to 500 MPa, preferably from 100 MPa to 500 MPa.

10. The process of claim 8, wherein the powder component of the bone cement formula contains a pore-forming agent, or a pore-forming agent is added during the mixing or is mixed with the paste before shaping the paste in a mold, and said process further comprises immersing said block of hardened bone cement composite in an immersing liquid to dissolve said pore-forming agent in the immersing liquid, creating pores therein, so that a porous block is formed, and preferably the porous block has a porosity of 50-90 vol%.

11. The process of claim 10, wherein the pore forming agent is selected from the group consisting of LiCl, KCI, NaCl, MgCl₂, CaCl₂, NalO₃, KI, Na₃PO₄, K₃PO₄, Na₂CO₃, amino acid-sodium salt, amino acid-potassium salt, glucose, polysaccharide, fatty acid-sodium salt, fatty acid-potassium salt, potassium bitartrate (KHC₄H₄O₆), potassium carbonate, potassium gluconate (KC₆H₁₁O₇), potassium-sodium tartrate (KNaC₄H₄O₆·4H₂O), potassium sulfate (K₂SO₄), sodium sulfate, sodium lactate and mannitol.

12. The process of claim 10, wherein the immersing liquid is a phosphate-containing solution having a phosphate concentration from 0.1 M to 6 M, preferably from 1 M to 3 M, or water.

13. The process of claims 8 or 10 further comprising impregnating the block of the porous block with an impregnating liquid for a period of time, so that a compressive strength of the resulting impregnated block or impregnated porous block removed from the impregnating liquid is increased compared to that of said block or said porous block without said impregnating treatment.

14. The process of claim 13, wherein the impregnating liquid is a phosphate-containing solution having a phosphate concentration from 0.1 M to 6 M, preferably from 1 M to 3 M.

15. The process of claims 8 or 10 further comprising breaking up the block or the porous block into pellets.

## Patentansprüche

1. Knochenzementformel, umfassend eine Pulverkomponente und eine Abbindeflüssigkeitskomponente mit einem Flüssigkeits-Pulver-Verhältnis von 0,20 ml/g (ccm/g) bis 0,50 ml/g (ccm/g), vorzugsweise 0,25 ml/g (ccm/g) bis 0,35 ml/g (ccm/g),
wobei die Pulverkomponente einen Calciumsulfatausgangsstoff und einen Calciumphosphatausgangsstoff mit einem Gewichtsverhältnis des Calciumsulfatausgangsstoffs von gröβer als 5 % und nicht größer als 55 %, bezogen auf das Gesamtgewicht des Calciumsulfatausgangsstoffs und des Calciumphosphatausgangsstoffs, umfasst,
wobei der Calciumsulfatausgangsstoff Calciumsulfathemihydrat (CSH), Calciumsulfatdehydrat (CSD) oder wasserfreies Calciumsulfat, und vorzugsweise CSH, ist, und der Calciumphosphatausgangsstoff Tetracalciumphosphat (TTCP) und Dicalciumphosphat in einem Molverhältnis von TTCP zu Dicalciumphosphat von 0,5 bis 2,5, vorzugsweise 1,0, umfasst, und
wobei die Abbindeflüssigkeitskomponente Ammoniumionen (NH₄⁺) in einer Konzentration von 0,5 M bis 4 M umfasst.

2. Formel nach Anspruch 1, wobei das Dicalciumphosphat wasserfreies Dicalciumphosphat (DCPA) ist.

3. Formel nach Anspruch 1, wobei die Abbindeflüssigkeitskomponente Ammoniumionen (NH₄⁺) in einer Konzentration von 1,0 M bis 2,0 M umfasst.

4. Formel nach Anspruch 3, wobei die Abbindeflüssigkeitskomponente eine Lösung von NH₄H₂PO₄, (NH₄)₂HPO₄, (NH₄)₃PO₄. 3H₂O oder einer Mischung von diesen ist.

5. Formel nach Anspruch 4, wobei die Abbindeflüssigkeitskomponente eine wässrige Lösung von (NH₄)₂HPO₄ ist.

6. Formel nach Anspruch 2, wobei der Calciumphosphatausgangsstoff aus TTCP und DCPA zusammengesetzt ist.

7. Formel nach Anspruch 1, außerdem umfassend ein porenbildendes Mittel, welches in einer Lösung gelöst werden soll, wenn ein daraus hergestellter gehärteter Knochenzementverbundstoff in die Lösung eingetaucht wird.

8. Verfahren zum Herstellen eines gehärteten Knochenzementverbundstoffs, umfassend das Bilden einer Knochenzementpaste durch Mischen der Pulverkomponente und der Abbindeflüssigkeitskomponente der Knochenzementformel, die in einem der Ansprüche 1 bis 6 angegeben ist; das Formen der Paste in einer Form; und das Entfernen der Form, um einen Block aus gehärtetem Knochenzementverbundstoff zu bilden.

9. Verfahren nach Anspruch 8, außerdem umfassend das Unterdrucksetzen der Paste in der Form, bevor die Paste abbindet, um einen Teil der Flüssigkeit aus der Paste zu entfernen, so dass ein Flüssigkeits-Pulver-Verhältnis der Paste abnimmt, wobei der an die Paste angelegte Druck in der Form von 1 MPa bis 500 MPa, vorzugsweise von 100 MPa bis 500 MPa beträgt.

10. Verfahren nach Anspruch 8, wobei die Pulverkomponente der Knochenzementformel ein porenbildendes Mittel enthält oder ein porenbildendes Mittel während des Mischens zugegeben wird oder mit der Paste vermischt wird, bevor die Paste in einer Form geformt wird, und das Verfahren außerdem das Eintauchen des Blocks aus gehärtetem Knochenzementverbundstoff in eine Eintauchflüssigkeit zum Auflösen des porenbildenden Mittels in der Eintauchflüssigkeit, wobei Poren darin erzeugt werden, umfasst, so dass ein poröser Block gebildet wird, und vorzugsweise der poröse Block eine Porosität von 50 - 90 Vol.-% aufweist.

11. Verfahren nach Anspruch 10, wobei das porenbildende Mittel ausgewählt ist aus der Gruppe bestehend aus LiCl, KCI, NaCl, MgCl₂, CaCl₂, NalO₃, KI, Na₃PO₄, K₃PO₄, Na₂CO₃, Aminosäure-Natriumsalz, Aminosäure-Kaliumsalz, Glucose, Polysaccharid, Fettsäure-Natriumsalz, Fettsäure-Kaliumsalz, Kaliumhydrogentartrat (KHC₄H₄O₆), Kaliumcarbonat, Kaliumgluconat (KC₆H₁₁O₇), Kalium-Natrium-Tartrat (KNaC₄H₄O₆ · 4H₂O), Kaliumsulfat (K₂SO₄), Natriumsulfat, Natriumlactat und Mannitol.

12. Verfahren nach Anspruch 10, wobei die Eintauchflüssigkeit eine phosphathaltige Lösung mit einer Phosphatkonzentration von 0,1 M bis 6 M, vorzugsweise von 1 M bis 3 M, oder Wasser ist.

13. Verfahren nach den Ansprüchen 8 oder 10, außerdem umfassend das Imprägnieren des Blocks oder des porösen Blocks mit einer Imprägnierungsflüssigkeit für einen Zeitraum, so dass eine Druckfestigkeit des resultierenden imprägnierten Blocks oder imprägnierten porösen Blocks, der aus der Imprägnierungsflüssigkeit herausgenommen wurde, im Vergleich zu der des Blocks oder des porösen Blocks ohne die Imprägnierungsbehandlung erhöht ist.

14. Verfahren nach Anspruch 13, wobei die Imprägnierungsflüssigkeit eine phosphathaltige Lösung mit einer Phosphatkonzentration von 0,1 M bis 6 M, vorzugsweise von 1 M bis 3 M ist.

15. Verfahren nach den Ansprüchen 8 oder 10, außerdem umfassend das Zerkleinern des Blocks oder des porösen Blocks zu Pellets.

## Revendications

1. Formule de ciment osseux comprenant un composant poudreux et un composant de prise liquide avec un ratio liquide/poudre de 0,20 ml/g (cm³/g) à 0,50 ml/g (cm³/g), de préférence de 0,25 ml/g (cm³/g) à 0,35 ml/g (cm³/g),
dans laquelle le composant poudreux comprend une source de sulfate de calcium et une source de phosphate de calcium avec un rapport massique de la source de sulfate de calcium supérieur à 5 % et inférieur ou égal à 55 %, sur base de la masse totale de la source de sulfate de calcium et de la source de phosphate de calcium,
dans laquelle la source de sulfate de calcium est un hémihydrate de sulfate de calcium (CSH, soit Calcium Sulfate Hemihydrate), un sulfate de calcium dehydraté (CSD, soit Calcium Sulfate Dehydrate) ou un sulfate de calcium anhydre, et de préférence un CSH, et la source de phosphate de calcium comprend du phosphate tétracalcique (TTCP, soit Tetracalcium Phosphate) et du phosphate dicalcique avec un rapport molaire de phosphate tétracalcique/dicalcique compris entre 0,5 et 2,5, et de préférence de 1,0, et
dans laquelle le composant de prise liquide comprend l'ion ammonium (NH₄⁺) avec une concentration de 0,5 M à 4 M.

2. Formule selon la revendication 1, dans laquelle le phosphate dicalcique est un phosphate dicalcique anhydre (DCPA, soit Dicalcium Phosphate Anhydrous).

3. Formule selon la revendication 1, dans laquelle le composant de prise liquide comprend l'ion ammonium (NH₄⁺) avec une concentration de 1,0 M à 2,0 M.

4. Formule selon la revendication 3, dans laquelle le composant de prise liquide est une solution de NH₄H₂PO₄, de (NH₄)₂HPO₄, de (NH₄)₃PO₄.3H₂O, ou d'un mélange de ceux-ci.

5. Formule selon la revendication 4, dans laquelle le composant de prise liquide est une solution aqueuse de (NH₄)₂HPO₄.

6. Formule selon la revendication 2, dans laquelle la source de phosphate de calcium est composée de TTCP et de DCPA.

7. Formule selon la revendication 1, comprenant en outre un agent de formation de pores qui doit être dissout dans une solution lorsqu'un composite de ciment osseux durci préparé à partir de ladite formule est immergé dans la solution.

8. Procédé de préparation d'un composite de ciment osseux durci, comprenant : la formation d'une pâte de ciment osseux en mélangeant le composant poudreux et le composant de prise liquide de la formule de ciment osseux selon l'une quelconque des revendications 1 à 6 ; la formation de la pâte dans un moule ; et l'enlèvement du moule pour former un bloc de composite de ciment osseux durci.

9. Procédé selon la revendication 8, comprenant en outre la mise sous pression de ladite pâte dans ledit moule avant que ladite pâte ne durcisse pour éliminer
une partie du liquide de ladite pâte, de manière à réduire le ratio liquide/poudre de ladite pâte,
dans lequel la pression appliquée à la pâte dans le moule est comprise entre 1 MPa et 500 MPa, et de préférence entre 100 MPa et 500 MPa.

10. Procédé selon la revendication 8, dans lequel le composant poudreux de la formule de ciment osseux contient un agent de formation de pores, ou dans lequel un agent de formation de pores est ajouté durant le mélange ou est mélangé avec la pâte avant la formation de la pâte dans un moule, et ledit procédé comprend en outre l'immersion dudit bloc de composite de ciment osseux durci dans un liquide d'immersion pour dissoudre ledit agent de formation de pores dans le liquide d'immersion, la création de pores dans ledit bloc de telle sorte qu'un bloc poreux est formé, le bloc poreux présentant de préférence une porosité de 50 % à 90 % en volume.

11. Procédé selon la revendication 10, dans lequel l'agent de formation de pores est sélectionné parmi le groupe constitué par LiCl, KCl, NaCl, MgCl₂, CaCl₂, NalO₃ , KI, Na₃PO₄, K₃PO₄, Na₂CO₃, un sel sodé d'acide aminé, un sel potassique d'acide aminé, le glucose, un polysaccharide, un sel sodé d'acide gras, un sel potassique d'acide gras, le bitartrate de potassium (KHC₄H₄O₆), le carbonate de potassium, le gluconate de potassium (KC₆H₁₁O₇), le tartrate de potassium-sodium (KNaC₄H₄O₆.4H₂O), le sulfate de potassium (K₂SO₄), le sulfate de sodium, le lactate de sodium et le mannitol.

12. Procédé selon la revendication 10, dans lequel le liquide d'immersion est une solution contenant du phosphate avec une concentration en phosphate comprise entre 0,1 M et 6 M, de préférence entre 1 M et 3 M, ou de l'eau.

13. Procédé selon la revendication 8 ou 10, comprenant en outre l'imprégnation du bloc ou du bloc poreux avec un liquide d'imprégnation durant une période donnée, de telle sorte que la résistance à la compression du bloc imprégné résultant ou du bloc poreux imprégné sorti du liquide d'imprégnation est augmentée en comparaison avec ledit bloc ou ledit bloc poreux sans ledit traitement d'imprégnation.

14. Procédé selon la revendication 13, dans lequel le liquide d'imprégnation est une solution contenant du phosphate et présentant une concentration en phosphate comprise entre 0,1 M et 6 M, et de préférence entre 1 M et 3 M.

15. Procédé selon la revendication 8 ou 10, comprenant en outre la fragmentation du bloc ou du bloc poreux en granulés.
